# EUROPEAN PATENT SPECIFICATION

(11) **EP 2 950 831 B1**
(45) Date of publication and mention of the grant of the patent: **12.09.2018**
(21) Application number: 14704175.0
(22) Date of filing: 28.01.2014
(51) Int. Cl.: A61L 27/08, A61K 9/00, A61K 9/06, A61L 24/00

(54) **CARBON NANOSTRUCTURES FOR OCULAR TISSUE REINFORCEMENT**
KOHLENSTOFFNANOSTRUKTUREN ZUR AUGENGEWEBEVERSTÄRKUNG
NANOSTRUCTURES DE CARBONE POUR LE RENFORCEMENT D'UN TISSU OCULAIRE

(30) Priority: 30.01.2013 US 201361758294 P
(43) Date of publication of application: 09.12.2015
(73) Proprietor: Vega Estrada, Alfredo, 03016 Alicante (ES); Alio Y Sanz, Jorge, 03016 Alicante (ES); Rodriguez Reinoso, Francisco, 03016 Alicante (ES); Silvestre Albero, Joaquin, 03016 Alicante (ES); Bataille, Laurent, 03016 Alicante (ES); Vissum Corporation S.L., 03016 Alicante (ES)
(72) Inventor: VEGA ESTRADA, Alfredo, 03016 Alicante (ES); ALIO Y SANZ, Jorge, 03016 Alicante (ES); RODRIGUEZ REINOSO, Francisco, E-03690 San Vicente del Raspeig (Alicante) (ES); SILVESTRE ALBERO, Joaquin, E-03690 San Vicente del Raspeig (Alicante) (ES); BATAILLE, Laurent, 03016 Alicante (ES)
(74) Representative: Carpintero Lopez, Francisco
(86) International application number: PCT/GB2014/050217
(87) International publication number: WO 2014/118522

(56) References cited:
- WO-A1-2009/018092
- WO-A1-2012/154677
- US-A1- 2011 287 067
- E. S. Avetisov ET AL: "Nonsurgical and surgical methods of sclera reinforcement in progressive myopia", Acta Ophthalmologica Scandinavica, vol. 75, no. 6, 1 December 1997 (1997-12-01), pages 618-623, XP55162265, ISSN: 1395-3907, DOI: 10.1111/j.1600-0420.1997.tb00617.x

## Description

### FIELD OF INVENTION

The present invention pertains to a composition and method for reinforcing biological tissues comprising a mixture of carbon nanotubes and graphene structures.

### BACKGROUND

Potential biological applications of carbon nanotubes (CNT) have further been explored.

The main difficulty of integrating such materials into biological systems derives from the difficulty to attain highly stable dispersions in physiological solutions. To extend the applications of carbon nanotubes in medicinal chemistry, water stable dispersions are in demand. It has been shown that carbon nanotubes can be dispersed in aqueous solution by a wrapping approach using starch and poly(vinylpyrrolidone) or attaching monoamine terminated poly(ethlyleneoxide), glucosamine or crown ethers to the carboxylic groups of the oxidized SWNTs.

The following prior art is believed to be the current status of the art:
PCT application No.2009018092 discloses nanoparticles, such as carbon nanotubes or other materials having extended aromatic surfaces (e.g., graphene sheet or nanotube), which are used to deliver active agents such as drugs, labels or dyes (termed for convenience a "drug") to the interior of cells. The nanoparticles are functionalized by a hydrophilic polymer to render them stable in suspension. This molecule may be covalently attached to the nanoparticle, or may be adsorbed thereto as an amphiphilic molecule. The nanoparticles are coupled to the drug through supramolecular bonding i.e., binding to the exterior of the nanoparticle through π - stacking. The drug may also be covalently bonded to the hydrophilic polymer, which is coupled to the nanoparticle through supramolecular bonding. The drug is therefore capable of release in the cell exterior. The drug is more rapidly released at lower pH, as found e.g., in tumor cells. The drug-coupled, functionalized nanoparticles may also be targeted to specific cells through modification of the hydrophilic polymer, e.g., by adding an RGD peptide, or an antibody, which is targeted to cells expressing integrins, or an antibody directed to a cell surface marker. The drug may also be linked to a branched chain hydrophilic polymer, so that each polymer molecule carries more than one drug bound by a cleavable linker.

US patent application No. 20060199770 relates to functionalized carbon nanotubes in medicinal chemistry and in immunology. The invention further describes a functionalized carbon nanotube, the surface of which carries covalently bound reactive and/or activable functional groups which are homogeneously distributed on the surface, the functionalized carbon nanotube being substantially intact and soluble in organic and/or aqueous solvents.

PCT application No.2012060592 relates to a coating film having a thickness of no more than 200 nm in which carbon nanotubes are mixed with a biocompatible polymer, and the invention is advantageous in that it has been confirmed that adjusting the synthesis proportions of the polymer and the carbon nanotubes can increase the biocompatibility and adjust the mechanical strength and can suppress toxicity and inflammation of the coating film having an ultra-thin-film thickness, and these characteristics can be used in articles of medical equipment which are inserted into microvessels in the body such as nano-sized medical devices and wire coatings.

PCT application No.2006116752 provides articles of manufacture comprising biocompatible nanostructures comprising nanotubes and nanopores for, e.g., organ, tissue and/or cell growth, e.g., for bone, kidney or liver growth, and uses thereof, e.g., for in vitro testing, in vivo implants, including their use in making and using artificial organs, and related therapeutics. The invention provides lock-in nanostructures comprising a plurality of nanopores or nanotubes, wherein the nanopore or nanotube entrance has a smaller diameter or size than the rest (the interior) of the nanopore or nanotube. The invention also provides dual structured biomaterial comprising micro- or macro-pores and nanopores.

E.S. Avetisov et al. disclose in "Nonsurgical and surgical methods of sclera reinforcement in progressive myopia" (Acta Ophthalmologica Sacndinavica 1997:75:618-623) a sclera strengthening injection in which a dose of liquid polymeric composition is injected under the Tenon's capsule on the scleral surface. After polymerization, the composition forms over the scleral surface a layer of elastic foamed gel to correct the biomechanical properties of sclera. The composition is a mixture of PVP, acrylamidehydrazide, ethylacrylate (1:1:1) in aqueous solution.

It is further known that Corneal ecstatic disorders are characterized by the weakening of the collagen fibers within the stroma of the cornea. Carbon nanomaterials (nanotubes and graphene) have demonstrated to increase the physical properties of different materials and different biological tissues.

It is further known that administration of medication for preventive or active inhibition of post-surgical complications is both problematic for the patient and ineffective due to toxicity associated with continued topical or systemic administration. Most drugs penetrate poorly through the cornea. The rate of corneal uptake is usually high initially but declines rapidly with additional exposure, a phenomenon leading to a transient period of overdose followed by an extended period of sub-therapeutic levels prior to the administration of the next scheduled dose.

It therefore remains a long felt and unmet need to provide novel means and methods using carbon nanotube compositions to provide an effective reinforcement of the biological tissues.

### SUMMARY

It is an object of the present invention to disclose a dispersion composition for reinforcing an ophthalmic tissue comprising: a mixture of carbon nanomaterials; and a physiological solution; wherein the carbon nonomaterial is carbon nanotubes (CNTs) mixed with a graphene structure selected from the group consisting of pure graphene, graphite oxide, reduced graphite oxide and a combination thereof.

It is an object of the present invention to provide the composition as defined above, wherein further comprising a biocompatible surfactant bound to the carbon nanomaterials.

It is another object of the present invention to provide the composition as defined above, wherein the carbon nanomaterials is nanotubes (CNTs) mixed with graphene structure; the CNTs and/or graphene structure are functionalized with surface groups. It is another object of the present invention to provide the composition as defined above, wherein the surfactant is selected from the group consisting of: Glucosamine hydrochloride, Chondroitrin Sulfate, Starch, and a combination thereof.

It is another object of the present invention to provide the composition as defined above, wherein the graphene structure is selected from the group consisting of: pure graphene, graphite oxide, reduced graphite oxide and a combination thereof.

It is another object of the present invention to provide the composition as defined above, wherein the composition is in a dosage form of drops, gel or mist.

It is another object of the present invention to provide the composition as defined above, wherein the composition is adapted as an adhesive material.

It is another object of the present invention to provide the composition as defined above, wherein the composition further adapted to facilitate the adhesion within the tissue, between different tissues or between the tissue and other biocompatible structures.

It is another object of the present invention to provide the composition as defined above, wherein the composition is mixed with a drug agent.

It is another object of the present invention to provide the composition as defined above, wherein the composition is applied directly to the tissue or by means of a pocket-like space to place the dispersion composition within the tissue.

It is another object of the present invention to provide the composition as defined above, wherein the carbon nanotubes are selected from the group consisting of: single wall nanotube (SWNT), double wall nanotube (DWNT), multiple wall carbon nanotube (MWCNT) and a combination thereof.

It is another object of the present invention to provide the composition as defined above, wherein the carbon nanotubes are combined with graphene structures in ratios ranging from about 0.01% to about 99.99%.

It is another object of the present invention to provide the composition as defined above, wherein the carbon nanotubes have an average length of about 0.05 microns to about 100 microns.

It is another object of the present invention to provide the composition as defined above, wherein the carbon nanotubes have an average diameter (OD) less than about 1nm It is another object of the present invention to provide the composition as defined above, wherein the carbon nanotubes have an average diameter (OD) of about 1nm to about 100 nm.

It is another object of the present invention to provide the composition as defined above, wherein the carbon nanotubes have a carbon purity more than about 60%

It is another object of the present invention to provide the composition as defined above, wherein the carbon nanotubes have a preferably carbon purity more than about 95%. It is another object of the present invention to provide the composition as defined above, wherein the graphene structure is at least a single carbon layer having a thickness of about 0.33 nm to about 1 nm.

It is another object of the present invention to provide the composition as defined above, wherein the graphene structure is a 2D graphene plane having dimensions in both directions from about 0.01 to 100 microns.

It is another object of the present invention to provide the composition as defined above, wherein the surfactant is bound to the carbon nanomaterials.

It is another object of the present invention to provide the composition as defined above, wherein the surfactant is selected from the group consisting of: monosaccharide, disaccharide, oligosaccharide, polysaccharide and combination thereof

It is another object of the present invention to provide the composition as defined above, wherein the surfactant is a drug composition selected from the group consisting of:
Ciclodextrin, Laurocapram, Benzalkonium chloride and combination thereof.

It is another object of the present invention to provide the composition as defined above, wherein the surfactant is selected from the group consisting of: amino sugar, amino acid, polyamino carboxylic acid and combination thereof.

It is another object of the present invention to provide the composition as defined above, wherein the surfactant is polyether such as polyethylene glycol.

It is another object of the present invention to provide the composition as defined above, wherein the surfactant covers at least 1% of the carbon nanomaterials surface.

It is another object of the present invention to provide the composition as defined above, wherein the physiological solution is a biocompatible solution such as aqueous solution, a saline solution or any other physiological solutions.

It is another object of the present invention to provide the composition as defined above, wherein the composition is further combined with ophthalmic viscosurgical device (OVD) in order obtained a better distribution within the ocular tissues.

It is another object of the present invention to provide the composition as defined above, wherein the composition is further combined with autologous blood derivative products such as platelet rich plasma or autolugus serum.

It is another object of the present invention to disclose a method of preparing a dispersion composition for reinforcing an ophthalmic tissue, comprising steps of:
(a)providing a dispersion composition comprising : a mixture of carbon nanomaterials; and, a physiological solution;
(b)obtaining the carbon mixture in dispersed form using the physiological solution; and,
(c)forming a stable suspension of the complex; wherein the step of providing the carbon nanomaterials comprises providing a mixture of carbon nanotubes (CNTs) and a graphene structure selected from the group consisting of pure graphene, graphite oxide, reduced graphite oxide and a combination thereof.

It is another object of the present invention to provide the method as defined above, wherein the method additionally comprising step of providing a biocompatible surfactant selected from the group consisting of: Dextran, Glucosamine hydrochloride, Chondroitrin Sulfate , Starch and a combination thereof.

It is another object of the present invention to provide the method as defined above, wherein the method additionally comprising step of attaching the surfactant to the carbon nanomaterials suspension.

It is another object of the present invention to provide the method as defined above, wherein the method additionally comprising step of sonicating the complex to improve the dispersion of the carbon nano-materials.

It is another object of the present invention to provide the method as defined above, wherein the method additionally comprising step of selecting the surface groups from the group consisting of: oxygen group, nitrogen group and a combination thereof.

It is another object of the present invention to provide the method as defined above, wherein the graphene structure is selected from the group consisting of: pure graphene, graphite oxide, reduced graphite oxide and a combination thereof.

It is another object of the present invention to provide the method as defined above, wherein the step of providing the composition as an adhesive material.

It is another object of the present invention to provide the method as defined above, wherein the step of providing the composition to facilitate the adhesion within the tissue, between different tissues or between the tissue and other biocompatible structures.

It is another object of the present invention to provide the method as defined above, wherein the method additionally comprising step of mixing the composition with a drug composition.

It is another object of the present invention to provide the method as defined above, wherein the method additionally comprising step of applying the composition directly to the tissue or by means of a pocket-like space for placing the dispersion composition within the tissue.

It is another object of the present invention to provide the method as defined above, wherein the method additionally comprising step of selecting the carbon nanotubes from the group consisting of: single wall nanotube (SWNT), double wall nanotube (DWNT), multiple wall carbon nanotube (MWCNT) and a combination thereof.

It is another object of the present invention to provide the method as defined above, wherein the method additionally comprising step of admixing the carbon nanotubes with graphene structures in ratios ranging from about 0.01% to about 99.99%.

It is another object of the present invention to provide the method as defined above, wherein the step pf providing carbon nanotubes having an average length of about 0.05 microns to about 100 microns.

It is another object of the present invention to provide the method as defined above, wherein the method additionally comprising step of providing the carbon nanotubes have an average diameter (OD) less than about 1nm.

It is another object of the present invention to provide the method as defined above, wherein the method additionally comprising step of providing the carbon nanotubes have an average diameter (OD) of about 1nm to about 100 nm.

It is another object of the present invention to provide the method as defined above, wherein the method additionally comprising step of providing the carbon nanotubes having a carbon purity more than about 60%.

It is another object of the present invention to provide the method as defined above, wherein the method additionally comprising step of providing the carbon nanotubes have a preferably carbon purity more than about 95%.

It is another object of the present invention to provide the method as defined above, wherein the graphene structure is at least one single carbon layer having a thickness of about 0.33 nm to about 1 nm.

It is another object of the present invention to provide the method as defined above, wherein the graphene structure is a 2D graphene planes having dimensions in both directions from about 0.01 to 100 microns.

It is another object of the present invention to provide the method as defined above, wherein the method additionally comprising step of providing the surfactant bounded to the carbon material.

It is another object of the present invention to provide the method as defined above, wherein the method additionally comprising step of selecting the surfactant from the group consisting of: monosaccharide, disaccharide, oligosaccharide, polysaccharide and a combination thereof.

It is another object of the present invention to provide the method as defined above, wherein the method additionally comprising step of providing the surfactant as a drug composition selected from the group consisting of: Ciclodextrin, Laurocapram, Benzalkonium chloride or combination thereof.

It is another object of the present invention to provide the method as defined above, wherein the method additionally comprising step of selecting the surfactant from the group consisting of: amino sugar, amino acid, polyamino carboxylic acid and a combination thereof.

It is another object of the present invention to provide the method as defined above, wherein the surfactant is polyether, preferably polyethylene glycol.

It is another object of the present invention to provide the method as defined above, wherein the step of providing the surfactant covering at least 1% of the carbon nanomaterials surface.

### BRIEF DESCRIPTION

In order to understand the invention and to see how it may be implemented in practice, a few preferred embodiments will now be described, by way of non-limiting example only, with reference to be accompanying drawings, in which:
**Fig. 1A** illustrates the creation of a pocket-like space within a corneal stroma ;
**Fig. 1B** illustrates a corneal storma comprising the composition of the present invention;

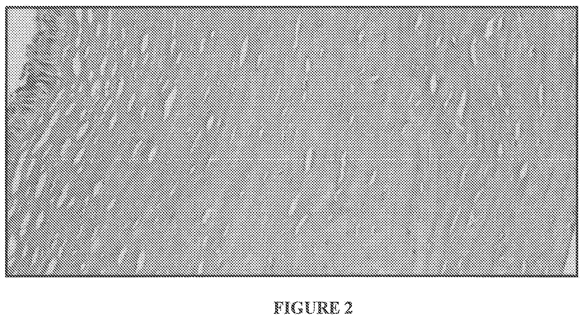

**Fig. 2** illustrates a corneal stroma sample with blue Alcian staining;
**Fig. 3** illustrates a corneal stroma sample with Masson trichrome staining;
**Fig. 4** illustrates a corneal stroma sample with Masson trichrome staining; and
**Fig. 5** illustrates the results of the biomechanical test.

### DETAILED DESCRIPTION

The following description is provided so as to enable any person skilled in the art to make use of the invention and sets forth the best modes contemplated by the inventor of carrying out this invention.

The carbon nanomaterial is carbon nanotubes (CNTs) mixed with pure graphene structures or with graphene structures containing surface functionalities such as oxygen surface groups, nitrogen surface groups or the like.The carbone nanotubes and/or the graphene may be functionalized.

The composition of the present invention further comprises a biocompatible surfactant. The biocompatible surfactant is selected from the group consisting of: Dextran, Glucosamine hydrochloride, Chondroitrin Sulfate , Starch and combination thereof.

The term *'biological tissues'* as used herein should be understood as ocular tissues such as cornea, sclera or the like.

Embodiments of the dispersion composition of the present invention comprise the graphene structures combined with carbon nanotubes in ratios ranging from about 0.01% to about 99.99%.

The dispersion composition may be in the form of drops, gel or mist.

In another embodiment of the present invention, the dispersion composition is aimed to reinforce biological tissues such as ophthalmic tissue or to facilitate the adhesion within the tissue, between different tissues or between the tissue and other biocompatible structures.

As used herein the term ***"about* X"** or ***"approximately* X"** or ***"substantially X"*** usually refers to a range 25% less than to 25% more than of X (X ± 25%), at times X ± 20%, X ± 15%, X ± 10% and preferably X ± 5%.

In another embodiment of the present invention, the dispersion composition used for reinforcing the ocular tissues can be further mixed with a drug delivery agent such as ciclodextrin or others drug delivery systems in order to achieve a better and effective distribution of the drug delivery systems within the tissue, therefore improving the quality, the surface properties and the performance of variety of drug delivery systems.

The drug delivery system is further used in order to obtain an adequate distribution of the dispersion composition of the present invention.

The drug delivery agent or system will further be adapted as a pharmaceutical vehicle to incorporate the carbon nanotubes and graphene structures within the tissue. The composition of the present invention further enhances the bioavailability of ocular medications.

The dispersion composition of the present invention may be used together with ophthalmic viscosurgical device (OVD) or with autologous blood derivative products in order to reinforce and obtained a better distribution within the ocular tissues.

The dispersion composition of the present invention may be used together with ophthalmic viscosurgical device (OVD) or with autologous blood derivative products in order to occlude tissue disruption (e.g. posterior capsular bag ropture) or to promote cell growing to occlude macular holes.

The dispersion composition of the present invention may be further combined with autologous blood derivative products such as platelet rich plasma or autolugus serum.

The dispersion composition of the present invention may be used to inhibit cell proliferation.

In another embodiment of the present invention, the dispersion composition may be used as an adhesive material. The dispersion composition may be applied directly to the tissue or by means of creating a pocket -like space in order to place the dispersion composition within the tissue.

In another embodiment of the present invention, the composition can be mixed with a drug composition.

The composition of the present invention can be combined with an ophthalmic drug conjugate which promotes diffusion and delivery from its carrier into the surrounding ocular environment.

Furthermore a pocket -like space may be created within the tissue either by manually dissecting the tissue with a surgical knife or by means of a femtosecond laser, such that the dispersion composition can be injected into the formed pocket -like space.

In another embodiment of the present invention, the dispersion composition used as an adhesive material can be directly applied to the surface of the tissue in order to generate a large contact area thus facilitating the adhesion within the tissue, between different tissues or between the tissue and other biocompatible structures.

The term *"Carbon nanotube"* is herein defined as a tube that contains a sheet of graphene rolled into a cylinder as small as 1 nm in diameter. Both single- walled nanotubes (SWNTs) and multiwalled nanotubes (MWNTs), with many concentric shells, have been synthesized. The nanotubes may further have electronic properties which depend upon the angle (and thus the chirality), with which it is rolled up. In terms of electrical properties, the nanotubes may be metallic, small-gap semiconductors, or large-gap semiconductors. Carbon nanotubes may include other materials. Metallic tubes have shown ballistic conduction on length scales of a micron or more. Carbon nanotubes, as used herein, includes structures that are not entirely carbon, such as BCN nanotubes. The present carbon nanotubes may also be graphene in other forms. This includes a single sheet of graphene formed into a sphere, which constitutes a carbon nanosphere, commonly referred to as a buckyball or fullerene. Sheets of graphene are also included.

The composition of the present invention comprises carbon nanotubes selected from the group consisting of: a single wall nanotube (SWNT), a double wall nanotube (DWNT), multiple wall carbon nanotube (MWCNT) and combination thereof.

The composition of the present invention preferably comprises single wall nanotube (SWNT).The carbon nanotubes have an average length of about 0.05 microns to about 100 microns. The carbon nanotubes have an average diameter (OD) less than 1nm.

The carbon nanotubes have an average diameter (OD) of about 1nm to about 100 nm. The carbon nanotubes have a carbon purity of greater than 60%, preferably carbon purity greater than 95%.

The term *"Graphene sheet"* herein refers to at least one- atom- thick two-dimensional layer of sp²-bonded carbon.

The terms graphene or grapheme are herein to be regarded as equivalent.

The composition of the present invention comprises graphene structures selected from the group consisting of: pure graphene, graphite oxide, reduced graphite oxide and combination thereof.

The graphene sheet of the present invention may incorporate graphite oxide, a layered material produced by the oxidation of graphite. In contrast to pristine graphite, the graphene-derived sheets in graphite oxide (graphene oxide sheets) are heavily oxygenated, bearing hydroxyl and epoxide functional groups on their basal planes, in addition to carbonyl and carboxyl groups located at the sheet edges. The presence of these functional groups makes graphene oxide sheets strongly hydrophilic, which allows graphite oxide to readily swell and disperse in water. The graphene structure is further a 2D graphene plane having dimensions in both directions from about 0.01 to several microns of about 100 microns.

The present invention comprises graphene structure which is at least a single carbon layer having a thickness of about 0.33 nm to about 1 nm. Several carbon layers may be formed.

The present carbon nanotubes may be made by a variety of processes including those made by arc discharge, laser ablation, chemical vapor deposition (CVD) and Hipco. Hipco is the High-pressure CO disproportionation process for catalytic production of SWNTs in a continuous-flow gas phase using CO as the carbon feedstock and Fe(CO)₅ as the iron- containing catalyst precursor. SWNTs are produced by flowing CO, mixed with a small amount of Fe(CO)₅, through a heated reactor. Size and diameter distribution of the nanotubes can be roughly selected by controlling the pressure of CO.

The composition of the present invention is preferably formulated in dosage unit form for ease of administration and uniformity of dosage.

The composition of the present invention comprising carbon nanotubes improves tracking of cells, sensing of microenvironments, delivering of transfection agents, and scaffolding for incorporating with the host's body. Carbon nanotubes may further be used for optical, magnetic resonance and radiotracer contrast agents in order to provide better means of evaluating tissue formation. Carbon nanotubes are further incorporated into scaffolds providing structural reinforcement as well as imparting novel properties such as electrical conductivity into the scaffolds may aid in directing cell growth. Potential cytotoxic effects associated with carbon nanotubes may be mitigated by chemically functionalizing the surface. Overall, carbon nanotubes may play an integral role as unique biomaterial for creating and monitoring engineered tissue.

There are several types of carbon nanotubes and related structures. Carbon nanotubes (CNTs) are classified as members of the fullerene family with a cylindrical nanostructure. Single-walled carbon nanotubes (SWCNTs) and multi-walled carbon nanotubes (MWCNTs) are the two primary types of CNTs. The composition of the present invention preferably comprises single-walled nanotubes (SWNTs) which have a diameter of at least 1 nanometer, with a tube length that can be many millions of times longer. The structure of a SWNT can be conceptualized by wrapping a one-atom-thick layer of graphite called graphene into a seamless cylinder. Multi-walled nanotubes (MWNT) consist of multiple rolled layers (concentric tubes) of graphene. Double-walled carbon nanotubes (DWNT) form a special class of nanotubes because their morphology and properties are similar to those of SWNT but their resistance to chemicals is significantly improved. This is especially important when functionalization is required thus, grafting of chemical functions at the surface of the nanotubes in order to add new properties to the CNT. In the case of SWNT, covalent functionalization will break C=C double bonds, leaving "holes" in the structure on the nanotube and, thus, modifying both its mechanical and electrical properties.

The SWCNTs, with a single layer of uniform graphite carbon (termed graphene) in a cylindrical conformation, typically have diameters between 0.5 nanometers to 2 nanometers and lengths greater than 1 nm.

The functionalized SWCNTs is useful for intracellular drug delivery since they have the ability to cross cell membranes. The hollow space comprising the interior of the nanotube can be filled with biologically important molecules. The SWCNTs target a specific cell or tissue type and act as biological cargo vehicles to transport and deliver therapeutic agents induced by a biochemical or biophysical stimulus. Furthermore, the SWCNTs themselves can be used as a therapeutic agent by exploiting their unique physical properties.

In another embodiment of the present invention, graphenated CNTs are a hybrid that combines graphitic foliates grown along the sidewalls of multiwalled or bamboo style CNTs. The fundamental advantage of an integrated graphene-CNT structure is the high surface area three-dimensional framework of the CNTs coupled with the high edge density of graphene. Graphene edges provide significantly higher charge density and reactivity than the basal plane, but they are difficult to arrange in a three-dimensional, high volume-density geometry. CNTs are readily aligned in a high density geometry (i.e., a vertically aligned forest) but lack high charge density surfaces; the sidewalls of the CNTs are similar to the basal plane of graphene and exhibit low charge density except where edge defects exist. Depositing a high density of graphene foliates along the length of aligned CNTs can significantly increase the total charge capacity per unit of nominal area as compared to other carbon nanostructures.

The external carbon sheath of the SWCNTs can be covalently or non-covalently functionalized with biological moieties that target specific cell or tissues types and/or pharmaceutical agents.

The present invention further comprises surfactant bound to the carbon material. The surfactant is selected from the group consisting of: monosaccharide, disaccharide, oligosaccharide, polysaccharide and combination thereof .The surfactant may be biocompatible.

The surfactant may further be a drug composition selected from the group consisting of: Ciclodextrin, Laurocapram, Benzalkonium chloride and combination thereof. Furthermore the surfactant is selected from the group consisting of: amino sugar, amino acid, polyamino carboxylic acid and combination thereof. The composition may further comprises biocompatible polyether surfactant such as polyethylene glycol. The surfactant covers at least 1% of the carbon nanomaterials surface.

The composition further comprises a physiological solution which is a biocompatible solution such as aqueous solution, a saline solution or any other biocompatible solutions.

The present invention further presents a method for preparing a dispersion composition for reinforcing an ophthalmic tissue, comprising the steps of:
(a) providing: (i) a mixture of carbon nanostructures, and (ii) a physiological solution.
(b) obtaining the carbon mixture in dispersed form using the physiological solution, and
(d) forming a stable suspension of the complex. The carbon nanotubes are preferably single-wall carbon nanotubes (SWCNTs) mixed with pure graphene structures or with functionalized graphene structures.

In another embodiment of the present invention the composition further comprises a biocompatible surfactant bound to the carbon nanomaterials. The surfactant is selected from the group consisting of: Dextran, Glucosamine hydrochloride, Chondroitrin Sulfate Starch and combination thereof. The surfactant is preferably Dextran.

In another embodiment of the present invention, the method further comprises the step of attaching a surfactant to the carbon nanomaterials suspension.

The method further comprises the step of sonicating the complex to improve the dispersion of the carbon nanomaterials.

In another embodiment of the present invention, the graphene structure is selected from the group consisting of: pure graphene, graphite oxide, reduced graphite oxide and combination thereof.

In another embodiment of the present invention, the composition is adapted as an adhesive material.

The composition further adapted to facilitate the adhesion within the tissue, between different tissues or between the tissue and other biocompatible structures.

In another embodiment of the present invention, the composition is mixed with a drug composition.

The functionalized SWCNTs is useful for intracellular drug delivery since they have the ability to cross cell membranes. The hollow space comprising the interior of the nanotube can be filled with biologically important molecules.

The SWCNTs target a specific cell or tissue type and act as biological cargo vehicles to transport and deliver therapeutic agents induced by a biochemical or biophysical stimulus. Furthermore, the SWCNTs themselves can be used as a therapeutic agent by exploiting their unique physical properties.

In another embodiment of the present invention, the composition is applied directly to the tissue or by means of a pocket-like space for placing the dispersion composition within the tissue.

In another embodiment of the present invention, the carbon nanotubes are selected from the group consisting of: single wall nanotubes (SWNT), double wall nanotubes (DWNT), multiple wall carbon nanotubes (MWCNT) and combination thereof.

In another embodiment of the present invention, the graphene structures combined with carbon nanotubes in ratios ranging from about 0.01% to about 99.99%.

In another embodiment of the present invention, the tubes have an average length of about 0.05 microns to about 100 microns.

In another embodiment of the present invention, the carbon nanotubes have an average diameter (OD) less than 1nm

In another embodiment of the present invention, the carbon nanotubes have an average diameter (OD) of about 1nm to about 100 nm.

In another embodiment of the present invention, the carbon nanotubes a carbon purity more than 60%

In another embodiment of the present invention, the carbon nanotubes have a preferably carbon purity more than 95%.

In another embodiment of the present invention, the graphene structure is at least a single carbon layer having a thickness of about 0.33 nm to about 1nm.

In another embodiment of the present invention, the graphene structure is a 2D graphene plane having dimensions in both directions from about 0.01 to 100 microns.

In another embodiment of the present invention, the surfactant is bounded to the carbon material.

In another embodiment of the present invention, the surfactant is selected from the group consisting of: monosaccharide, disaccharide, oligosaccharide, polysaccharide and combination thereof

In another embodiment of the present invention, the surfactant is a drug composition selected from the group consisting of: Ciclodextrin, Laurocapram, Benzalkonium chloride or combination thereof.

In another embodiment of the present invention, the surfactant is selected from the group consisting of: amino sugar, amino acid, polyamino carboxylic acid and combination thereof.

In another embodiment of the present invention, the surfactant is polyether preferably polyethylene glycol.

In another embodiment of the present invention, the surfactant covers at least 1% of the carbon nanomaterials surface .

### Examples

An experimental protocol was carried out where 24 rabbit eyes models were included. The population under study was divided into 4 different groups:
**Group 1** (Control group): 5 eye models without surgery.

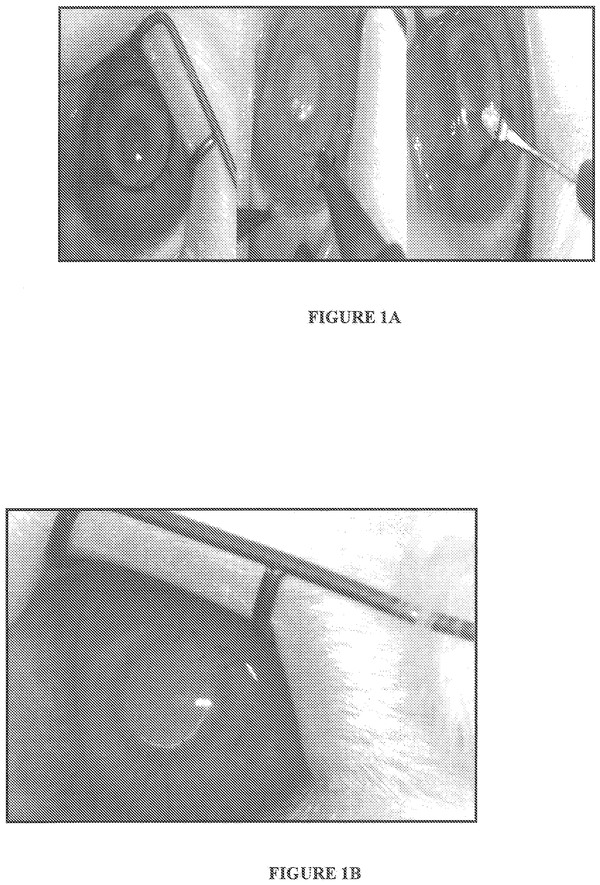

Reference is now made to **Fig. 1A** which presents **Group 2**(Pocket group): Fig. 1A illustrates a pocket-like space which was formed in the middle of the corneal stroma. A circle of 6mm diameter was marked on the surface of the cornea. Then a vertical incision with a depth of about *ca.* 300 microns was formed using a calibrated diamond knife. Finally, the dissection of the corneal stroma through an area of 6mm and at 300 microns in depth was preformed using a minicrescent surgical knife

Reference is now made to **Fig 1B** which presents **Group 3**(Reference 1 Group); Fig 1B presents eye models in which a pocket was created and a dispersion composed of carbon nanotubes (CNT) mixed with saline solution was injected inside the pocket-like space. The concentration of the composition is 0.1mg/ml.

**Group 4** (Reference 2 Group): eyes models in which a pocket was formed and a dispersion composed of carbon nanotube (CNT) mixed with saline solution was injected inside the pocket.

Reference is now made to **Fig 1B** which presents the cornea comprising the dispersion composition of the present invention comprising carbon nanotube (CNT) mixed with saline solution. After the injection of the composition containing CNTs, the eye models were kept under observation for a period of 3 months. Afterwards euthanasia was performed in the rabbits and the eyes enucleated. The safety and biocompatibility of the CNTs dispersion composition in the corneal tissue was assessed by means of pathology examination of the samples 3 months after the surgery.

The dispersion composition of the present invention, enhanced with Graphene and a surfactant agent has improved transparency properties in comparison to composition comprising CNT/saline solution with no additional surfactant agent.

Stress-strain measurements was carried out which further determine the modulus of elasticity of the corneal tissue therefore the mechanical properties of the cornea. Furthermore a strip of corneal tissue was dissected with a dimension of 5x20mm which further was clamped into a biomaterial tester. The Young's modulus was calculated in megapascales (MPa) in order to assess the mechanical properties of the samples.

### Pathology evaluation

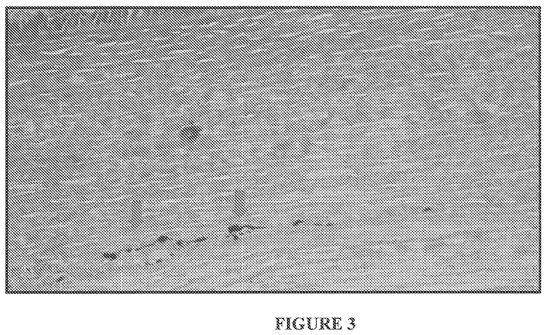

Reference is now made to **Fig. 3** shows corneal stroma sample with Masson trichrome. The solid red arrows indicates the carbone nantubes within the corneal tissue.

It is further illustrated that there are no signs of active inflammation after the procedure. The latest is corroborated when the Masson trichrome staining is performed.

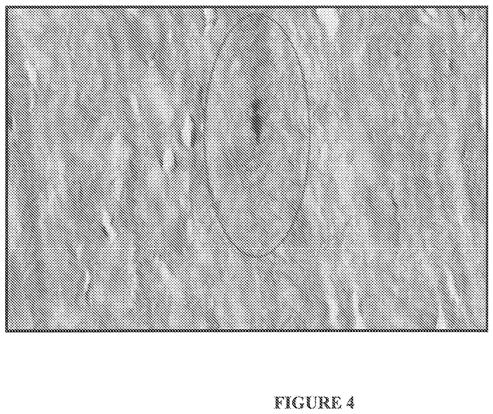

Reference is now made to **Fig. 4** which presents a corneal stroma sample with Masson trichrome staining, whilst the red circle indicates the strong adhesion of the tissue with the CNTs. It is further presented the sample of corneal stroma with blue Alcian staining which illustrates that there is no fibrous scaring and no alterations in the mucopolysaccharides of the corneal stroma.

**Fig. 4** illustrates another sample with Masson trichrome staining to show that there is no inflammation and no foreign body giant cell reaction against the CNT implanted. It also shows a stronger adhesion of the tissue in the area surrounding the CNT. Other interesting findings that were observed in the pathology report were the absence of neovascularization and that there were no apoptosis of the keratocytes on the samples that were examined.

### Biomechanical evaluation;

**Table 1**

| **Group** | **Young's modulus (MPa)** |
|---|---|
| **CONTROL** | **12,1+_2,5** |
| **POCKET** | **12,0+_4,2** |
| **REFERENCE 1** | **11,8+_1,3** |
| **REFERENCE 2** | **13,0+_3,9** |

Table 1 presents the Young's modulus obtained in each one of the samples. The Young modulus also known as the tensile modulus or elastic modulus, which measure of the stiffness of a material and further to characterize the material. The Young modulus presents the ratio of the uniaxial stress over the uniaxial strain in the range of stress in which Hooke's law holds. The table illustrates a trend towards a higher rigidity in the sample Reference 2 (CNT 1mg/ml), although these difference were not statistically significant.

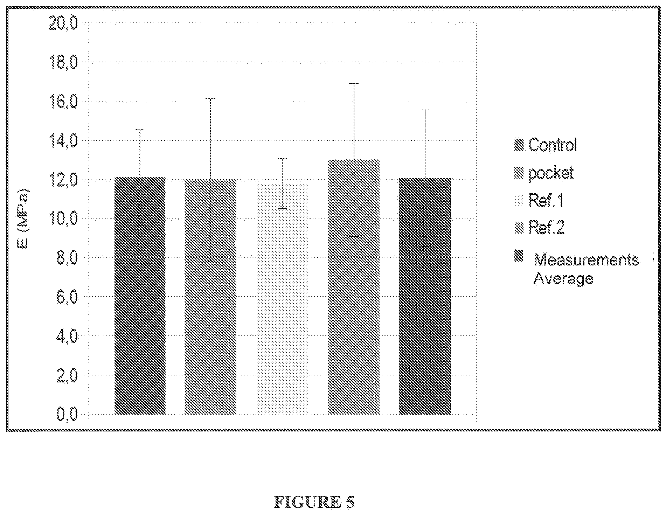

Reference is now made to **Fig. 5** which presents the results of biomechanical evaluation. The experimental protocol further confirms that carbon nanotubes are biocompatible with the corneal tissue as it is shown by the pathology evaluation.

Corneal ecstatic disorders are characterized by the weakening of the collagen fibers within the stroma of the cornea. The experimental protocol demonstrates that carbon nanomaterials such as carbon nanotubes and graphene increase the physical properties of different materials and different biological tissues.

Applying the CNTs of the present invention to the corneal tissue is a safe procedure as these carbon nanostructures does not induce fibrous scaring, foreign body giant cell reaction, neovascularization or apoptosis of the cells.

The Pathology evaluation further showed a strong adhesion between the corneal collagen fibers and the CNTs.

The Biomechanical evaluation shows that there is a trend towards more rigid corneal tissue after CNT implantation, although these changes were not statistically significant. The latest can be due to the small sample analyzed in the current experiment, or because a better distribution of the material along the tissue is necessary to obtained a higher reinforcement of the corneal stroma. A better dispersion of the CNT can be achieved using drug delivery molecules (e.g., Ciclodextrin, Laurocapram, Benzalkonium chloride) or other drug delivery systems. Further experimental protocols using these molecules are being performed to confirm this hypothesis. We also hypothesized that using other carbon nanomaterials, such as graphene, or a mixture of graphene/CNTs composition will induce a further reinforcement of the tissue in which the composition is applied.

Enhancement of the dispersion will lead to a more transparent composition. The present experiment, the dispersion was composed of just carbon nanotubes and saline solution without any surfactant. Other carbon nanostructures, such as graphene, or a mixture of graphene/CNTs, may be adapted together with surfactants, furthermore the transparency of the dispersion composition can be improved

The carbon nanostructures are biocompatible and safe for the corneal stroma. Other experements have also shown that carbon nanostructures have the ability to increase the rigidity of other materials such as construction, textile, electronic, biology and other industry materials and other structures composed of collagen fibers. Thus, the carbon nanomaterials reinforce the corneal collagen fibers and other tissues of the eye globe.

## Claims

1. A dispersion composition for reinforcing an ophthalmic tissue comprising:
a. a mixture of carbon nanomaterials; and
b. a physiological solution;
wherein said carbon nanomaterial is carbon nanotubes (CNTs) mixed with a graphene structure selected from the group consisting of pure graphene, graphite oxide, reduced graphite oxide and a combination thereof.

2. The composition according to claim 1, further comprising a biocompatible surfactant bound to the carbon nanomaterials.

3. The composition according to claim 2, wherein said surfactant is selected from the group consisting of: glucosamine hydrochloride, chondroitrin sulfate, starch, monosaccharide, disaccharide, oligosaccharide, polysaccharide, amino sugar, amino acid, polyamino carboxylic acid and any combination thereof.

4. The composition according to claim 2, wherein said surfactant is a drug composition selected from the group consisting of: cyclodextrin, laurocapram, benzalkonium chloride and any combination thereof.

5. The composition according to claim 2, wherein said surfactant is a polyether, said polyether is polyethylene glycol.

6. The composition according to claim 2, wherein said surfactant covers at least 1% of the carbon nanomaterial's surface.

7. The composition according to claim 1, wherein one of the following holds true:
a. said CNTs and/or graphene structure are functionalized with surface groups;
b. said composition is in a dosage form selected from the group consisting of drops, gel and mist;
c. said composition further adapted to facilitate the adhesion within the tissue, between different tissue, or between the tissue and other biocompatible structures;
d. said composition is mixed with a drug agent; and
e. said composition is adapted to be applied in a manner selected from the group consisting of: directly to the tissue; and by means of a pocket-like space to place said dispersion composition within said tissue.

8. The composition according to claim 1, wherein one of the following holds true:
a. said carbon nanotubes are selected from the group consisting of: single wall nanotube (SWNT), double wall nanotube (DWNT), multiple wall carbon nanotube (MWCNT) and a combination thereof;
b. the fraction of carbon nanotubes in said mixture of carbon nanomaterials is between 0.01% and 99.99%;
c. said carbon nanotubes are **characterized by** an average length lying between 0.05 µm and 100 µm;
d. said graphene structure is a 2D graphene plane having dimensions in both directions from 0.01 to 100 µm;
e. said carbon nanotubes are **characterized by** an average diameter (OD) of less than 1 nm; and
f. said carbon nanotubes are **characterized by** an average diameter (OD) lying between 1 nm and 100 nm.

9. The composition according to claim 1, wherein one of the following holds true:
a. said carbon nanotubes have a carbon purity of more than 60%;
b. said graphene structure is at least a single carbon layer having a thickness of between 0.33 nm and 1 nm;
c. said physiological solution is a biocompatible saline solution,
d. said composition is further combined with ophthalmic viscosurgical device (OVD) in order to obtain a better distribution within the ocular tissues; and
e. said composition is further combined with at least one autologous blood derivative products; said autologous blood derivative product is selected from the group consisting of platelet rich plasma or autologous serum.

10. A method of preparing a dispersion composition for reinforcing an ophthalmic tissue comprising:
a. providing a dispersion composition comprising :
i. a mixture of carbon nanomaterials; and
ii. a physiological solution;
b. obtaining said carbon mixture in dispersed form using said physiological solution; and
c. forming a stable suspension of the complex;
wherein said step of providing a mixture of carbon nanomaterials comprises providing a mixture of carbon nanotubes (CNTs) and a graphene structure selected from the group consisting of pure graphene, graphite oxide, reduced graphite oxide and a combination thereof.

11. The method according to claim 10, wherein one of the following holds true:
a. additionally comprising providing a biocompatible surfactant;
said surfactant is selected from the group consisting of: dextran, glucosamine hydrochloride, chondroitrin sulfate, starch, monosaccharide, disaccharide, oligosaccharide, polysaccharide, amino sugar, amino acid, polyamino carboxylic acid and any combination thereof;
b. additionally comprising binding said surfactant to said carbon nanomaterial;
c. additionally comprising providing said surfactant as a drug composition selected from the group consisting of cyclodextrin, laurocapam, benzalkonium chloride and any combination thereof; and
d. said surfactant is a polyether; said polyether is polyethylene glycol.

12. The method according to claim 11, wherein said step of providing said surfactant covering at least 1% of the carbon nanomaterial's surface.

13. The method according to claim 10, wherein one of the following holds true:
a. additionally comprising functionalizing said CNTs and/or said graphene material with surface groups;
b. additionally comprising selecting said at least one surface group from the group consisting of oxygen groups, nitrogen groups and a combination thereof;
c. additionally comprising providing said composition as an adhesive material;
d. additionally comprising providing said composition to facilitate the adhesion within the tissue, between different tissues, or between the tissue and other biocompatible structures; and
e. additionally comprising mixing said composition with a drug composition.

14. The method according to claim 10, wherein one of the following holds true:
a. additionally comprising selecting said carbon nanotubes from the group consisting of single wall nanotube (SWNT), double wall nanotube (DWNT), multiple wall carbon nanotube (MWCNT) and a combination thereof;
b. said step of providing carbon nanotubes comprises providing carbon nanotubes having an average length of between 0.05 µm and 100 µm;
c. said step of providing carbon nanotubes comprises providing carbon nanotubes having an average diameter (OD) of less than 1 nm;
d. said step of providing carbon nanotubes comprises providing carbon nanotubes having an average diameter (OD) of between 1 nm and 100 nm; and
e. said step of providing carbon nanotubes comprises providing carbon nanotubes having a carbon purity of greater than 60%.

15. The method according to claim 10, wherein one of the following holds true:
a. additionally comprising admixing said carbon nanotubes with graphene structures in ratios ranging from 0.01% to 99.99%; and
b. additionally comprising sonicating said complex to improve the dispersion of the carbon nanomaterials.

## Patentansprüche

1. Dispersionszusammensetzung zum Verstärken eines ophthalmischen Gewebes, umfassend:
a. eine Mischung aus Kohlenstoff-Nanomaterialien; und
b. eine physiologische Lösung;
wobei das Kohlenstoff-Nanomaterial aus Kohlenstoff-Nanoröhren (CNTs) besteht, die mit einer Graphenstruktur gemischt sind, die ausgewählt ist aus einer Gruppe bestehend aus reinem Graphen, Graphitoxid, reduziertem Graphitoxid und einer Kombination davon.

2. Zusammensetzung gemäß Anspruch 1, ferner umfassend ein biokompatibles Tensid, das an die Kohlenstoff-Nanomaterialien gebunden ist.

3. Zusammensetzung gemäß Anspruch 2, wobei das Tensid ausgewählt ist aus der Gruppe bestehend aus: Glucosaminhydrochlorid, Chondroitrinsulfat, Stärke, Monosaccharid, Disaccharid, Oligosaccharid, Polysaccharid, Aminozucker, Aminosäure, Polyaminocarbonsäure und jeglicher Kombination davon.

4. Zusammensetzung gemäß Anspruch 2, wobei das Tensid eine Arzneimittelzusammensetzung ist, ausgewählt aus der Gruppe bestehend aus Cyclodextrin, Laurocapram, Benzalkoniumchlorid und jeglicher Kombination davon.

5. Zusammensetzung gemäß Anspruch 2, wobei das Tensid ein Polyether ist, wobei das Polyether Polyethylenglycol ist.

6. Zusammensetzung nach Anspruch 2, wobei das Tensid mindestens 1% der Oberfläche des Kohlenstoff-Nanomaterials bedeckt.

7. Zusammensetzung gemäß Anspruch 1, wobei einer der folgenden Punkte gilt:
a. die CNTs und/oder die Graphenstruktur sind mit Oberflächengruppen funktionalisiert;
b. die Zusammensetzung ist in einer Dosierungsform ausgewählt aus der Gruppe bestehend aus Tropfen, Gel oder Nebel;
c. die Zusammensetzung ist weiterhin dazu ausgebildet, die Adhäsion innerhalb des Gewebes, zwischen verschiedenen Geweben oder zwischen dem Gewebe und anderen biokompatiblen Strukturen zu erleichtern;
d. die Zusammensetzung ist mit einem Arzneimittel vermischt; und
e. die Zusammensetzung ist ausgebildet, auf eine Art und Weise aufgebracht zu werden, die aus der Gruppe ausgewählt wurde, die besteht aus: direkt auf das Gewebe; und über einen taschenartigen Raum, um die Dispersionszusammensetzung innerhalb des Gewebes zu platzieren.

8. Zusammensetzung gemäß Anspruch 1, wobei einer der folgenden Punkte gilt:
a. die Kohlenstoff-Nanoröhren sind ausgewählt aus der Gruppe bestehend aus: einwandige Nanoröhre (SWNT), doppelwandige Nanoröhre (DWNT), mehrwandige Kohlenstoff-Nanoröhre (MWCNT) und einer Kombination davon;
b. der Anteil der Kohlenstoff-Nanoröhren in der Mischung der Kohlenstoff-Nanomaterialien ist zwischen 0,01% und 99,99%;
c. die Kohlenstoff-Nanoröhren sind durch eine durchschnittliche Länge zwischen 0,05 µm und 100 µm gekennzeichnet;
d. die Graphenstruktur ist eine 2D-Graphenebene mit Abmessungen in beiden Richtungen von 0,01 bis 100 µm;
e. die Kohlenstoff-Nanoröhren sind durch einen durchschnittlichen Durchmesser (OD) von weniger als 1 nm gekennzeichnet; und
f. die Kohlenstoff-Nanoröhren sind durch einen durchschnittlichen Durchmesser (OD) zwischen 1 nm und 100 nm gekennzeichnet.

9. Zusammensetzung nach Anspruch 1, wobei einer der folgenden Punkte gilt:
a. die Kohlenstoff-Nanoröhren weisen eine Kohlenstoffreinheit von mehr als 60% auf;
b. die Graphenstruktur ist mindestens eine einzelne Kohlenstoffschicht mit einer Dicke zwischen 0,33 nm und 1 nm;
c. die physiologische Lösung ist eine biokompatible salzhaltige Lösung
d. die Zusammensetzung ist ferner mit einem Viskoelastikum (OVD: Ophthalmic Viscosurgical Device) kombiniert, um eine bessere Verteilung innerhalb der Augengewebe zu erhalten; und
e. die Zusammensetzung ist ferner mit mindestens einem autologen Blutderivatprodukt kombiniert ist, wobei das autologe Blutderivatprodukt ausgewählt ist aus der Gruppe bestehend aus blutplättchenreichem Plasma oder autologem Serum.

10. Verfahren zur Herstellung einer Dispersionszusammensetzung zur Verstärkung eines ophthalmischen Gewebes, umfassend:
a. Bereitstellen einer Dispersionszusammensetzung, umfassend:
i. eine Mischung aus Kohlenstoff-Nanomaterialien; und
ii. eine physiologische Lösung;
b. Erhalten der Kohlenstoffmischung in dispergierter Form unter Verwendung der physiologischen Lösung; und
c. Bildung einer stabilen Suspension des Komplexes;
wobei der Schritt des Bereitstellens der Kohlenstoff-Nanomaterialien das Bereitstellen einer Mischung von Kohlenstoff-Nanoröhren (CNTs) und einer Graphenstruktur umfasst, die ausgewählt ist aus der Gruppe bestehend aus reinem Graphen, Graphitoxid, reduziertem Graphitoxid und einer Kombination davon.

11. Verfahren gemäß Anspruch 10, wobei einer der folgenden Punkte gilt:
a. zusätzlich umfassend den Schritt des Bereitstellens eines biokompatiblen Tensids;
wobei das Tensid ausgewählt ist aus der Gruppe bestehend aus: Dextran, Glucosaminhydrochlorid, Chondroitrinsulfat, Stärke, Monosaccharid, Disaccharid, Oligosaccharid, Polysaccharid, Aminozucker, Aminosäure, Polyaminocarbonsäure und jeglicher Kombination davon;
b. zusätzlich umfassend den Schritt des Bindens des Tensids an das Kohlenstoff-Nanomaterial;
c. zusätzlich umfassend den Schritt des Bereitstellens des Tensids als Arzneimittelzusammensetzung, ausgewählt aus der Gruppe bestehend aus Cyclodextrin, Laurocapram, Benzalkoniumchlorid und jeglicher Kombination davon; und
d. das Tensid ist ein Polyether, wobei der Polyether Polyethylenglycol ist.

12. Verfahren gemäß Anspruch 11, wobei nach dem Schritt des Bereitstellens des Tensids dieses mindestens 1% der Kohlenstoff-Nanomaterialoberfläche bedeckt.

13. Verfahren nach Anspruch 10, wobei einer der folgenden Punkte gilt:
a. zusätzlich umfassend den Schritt des Funktionalisierens der CNTs und/oder des Graphenmaterials mit Oberflächengruppen;
b. zusätzlich umfassend den Schritt des Auswählens der mindestens einen Oberflächengruppe aus der Gruppe bestehend aus Sauerstoffgruppen, Stickstoffgruppen und einer Kombination davon;
c. zusätzlich umfassend den Schritt des Bereitstellens der Zusammensetzung als ein Klebematerial;
d. zusätzlich umfassend den Schritt des Bereitstellens der Zusammensetzung, um die Adhäsion innerhalb des Gewebes, zwischen verschiedenen Geweben oder zwischen dem Gewebe und anderen biokompatiblen Strukturen zu erleichtern; und
e. zusätzlich umfassend den Schritt des Vermischens der Zusammensetzung ist mit einer Arzneimittelzusammensetzung.

14. Verfahren nach Anspruch 10, wobei einer der folgenden Punkte gilt:
a. zusätzlich umfassend den Schritt des Auswählens der Kohlenstoff-Nanoröhren aus der Gruppe bestehend aus: einwandige Nanoröhre (SWNT), doppelwandige Nanoröhre (DWNT), mehrwandige Kohlenstoff-Nanoröhre (MWCNT) und einer Kombination davon;
b. der Schritt des Bereitstellens der Kohlenstoff-Nanoröhren umfasst das Bereitstellen von Kohlenstoff-Nanoröhren mit einer durchschnittlichen Länge zwischen 0,05 µm und 100 µm;
c. der Schritt des Bereitstellens der Kohlenstoff-Nanoröhren umfasst das Bereitstellen der Kohlenstoff-Nanoröhren mit einem durchschnittlichen Durchmesser (OD) von weniger als 1 nm;
d. der Schritt des Bereitstellens der Kohlenstoff-Nanoröhren umfasst das Bereitstellen der Kohlenstoff-Nanoröhren mit einem durchschnittlichen Durchmesser (OD) zwischen 1 nm und 100 nm, und
e. der Schritt des Bereitstellens der Kohlenstoff-Nanoröhren umfasst das Bereitstellen von Kohlenstoff-Nanoröhren mit einer Kohlenstoffreinheit von mehr als 60%.

15. Verfahren nach Anspruch 10, wobei einer der folgenden Punkte gilt:
a. zusätzlich umfassend den Schritt des Zumischens der Kohlenstoff-Nanoröhren mit Graphenstrukturen in Verhältnissen im Bereich von 0,01% bis 99,99%; und
b. zusätzlich umfassend den Schritt der Ultraschallbehandlung des Komplexes, um die Dispersion des Kohlenstoff-Nanomaterials zu verbessern.

## Revendications

1. Composition de dispersion pour renforcer un tissu ophtalmique comprenant :
a. un mélange de nanomatériaux de carbone ; et
b. une solution physiologique ;
dans laquelle ledit nanomatériau de carbone est des nanotubes de carbone (NTC) mélangés à une structure de graphène choisie parmi le groupe constitué de graphène pur, d'oxyde de graphite, d'oxyde de graphite, d'oxyde de graphite réduit et d'une combinaison de ceux-ci.

2. Composition selon la revendication 1, comprenant en outre un tensioactif biocompatible lié aux nanomatériaux de carbone.

3. Composition selon la revendication 2, dans laquelle ledit tensioactif est choisi parmi le groupe constitué de : chlorhydrate de glucosamine, sulfate de chondroïtine, amidon, monosaccharide, disaccharide, oligosaccharide, polysaccharide, sucre aminé, acide aminé, acide polyamino carboxylique et toute combinaison de ceux-ci.

4. Composition selon la revendication 2, dans laquelle ledit tensioactif est une composition pharmaceutique choisie parmi le groupe constitué de : cyclodextrine, laurocapram, chlorure de benzalkonium et toute combinaison de ceux-ci.

5. Composition selon la revendication 2, dans laquelle ledit tensioactif est un polyéther, ledit polyéther est du polyéthylène glycol.

6. Composition selon la revendication 2, dans laquelle ledit tensioactif couvre au moins 1% de la surface du nanomatériau de carbone.

7. Composition selon la revendication 1, dans laquelle l'une des caractéristiques suivantes est présente :
a. lesdites NTC et/ou structure de graphène sont fonctionnalisés avec des groupes de surface ;
b. ladite composition se présente sous une forme galénique choisie parmi le groupe constitué par des gouttes, du gel et de la brume ;
c. ladite composition est en outre adaptée pour faciliter l'adhésion à l'intérieur du tissu, entre différents tissus ou entre le tissu et d'autres structures biocompatibles ;
d. ladite composition est mélangée à un agent pharmaceutique ; et
e. ladite composition est adaptée pour être appliquée d'une manière choisie parmi le groupe consistant en : directement sur le tissu ; et au moyen d'un espace en forme de poche pour placer ladite composition de dispersion à l'intérieur dudit tissu.

8. Composition selon la revendication 1, dans laquelle l'une des caractéristiques suivantes est présente :
a. lesdits nanotubes de carbone sont choisis parmi le groupe constitué de : nanotube à paroi simple (SWNT), nanotube à double paroi (DWNT), nanotube de carbone à parois multiples (MWCNT) et une combinaison de ceux-ci ;
b. la fraction de nanotubes de carbone dans ledit mélange de nanomatériaux de carbone est comprise entre 0,01 % et 99,99 % ;
c. lesdits nanotubes de carbone sont **caractérisés par** une longueur moyenne comprise entre 0,05 µm et 100 µm ;
d. ladite structure de graphène est un plan de graphène 2D ayant des dimensions dans les deux directions de 0,01 à 100 µm ;
e. lesdits nanotubes de carbone sont **caractérisés par** un diamètre moyen (OD) inférieur à 1 nm ; et
f. lesdits nanotubes de carbone sont **caractérisés par** un diamètre moyen (OD) compris entre 1 nm et 100 nm.

9. Composition selon la revendication 1, dans laquelle l'une des caractéristiques suivantes est présente :
a. lesdits nanotubes de carbone ont une pureté de carbone supérieure à 60%;
b. ladite structure de graphène est au moins une seule couche de carbone ayant une épaisseur comprise entre 0,33 nm et 1 nm ;
c. ladite solution physiologique est une solution saline biocompatible ;
d. ladite composition est en outre combinée avec un dispositif viscochirurgical ophtalmique (OVD) en afin d'obtenir une meilleure répartition au sein des tissus oculaires ; et
e. ladite composition est en outre combinée avec au moins un dérivé sanguin autologue. produits ; ledit produit dérivé sanguin autologue est choisi parmi le groupe composé de plasma riche en plaquettes ou de sérum autologue.

10. Procédé de préparation d'une composition de dispersion pour renforcer un tissu ophtalmique comprenant :
a. la fourniture d'une composition de dispersion comprenant :
i. un mélange de nanomatériaux de carbone ; et
ii. une solution physiologique ;
b. l'obtention dudit mélange de carbone sous forme dispersée en utilisant ladite solution physiologique ; et
c. la formation d'une suspension stable du complexe ;
dans laquelle ladite étape de fourniture d'un mélange de nanomatériaux de carbone comprend la fourniture d'un mélange de nanotubes de carbone (NTC) et d'une structure de graphène choisie parmi le groupe constitué de graphène pur, d'oxyde de graphite, d'oxyde de graphite réduit et d'une combinaison de ceux-ci.

11. Procédé selon la revendication 10, dans lequel l'une des caractéristiques suivantes est présente :
a. comprenant en outre la fourniture d'un tensioactif biocompatible ; ledit tensioactif est choisi parmi le groupe constitué de : dextrane, chlorhydrate de glucosamine, sulfate de chondroïtine, amidon, monosaccharide, disaccharide, oligosaccharide, polysaccharide, sucre aminé, acide aminé, acide polyamino carboxylique et toute combinaison de ceux-ci ;
b. comprenant en outre la liaison dudit tensioactif audit nanomatériau de carbone ;
c. comprenant en outre la fourniture dudit tensioactif sous la forme d'une composition pharmaceutique choisie parmi le groupe constitué par la cyclodextrine, le laurocapam, le chlorure de benzalkonium et toute combinaison de ceux-ci ; et
d. ledit agent tensioactif est un polyéther ; ledit polyéther est le polyéthylène glycol.

12. Procédé selon la revendication 11, dans lequel ladite étape de fourniture dudit tensioactif recouvre au moins 1% de la surface du nanomatériau de carbone.

13. Procédé selon la revendication 10, dans lequel l'une des caractéristiques suivantes est présente :
a. comprenant en outre la fonctionnalisation desdits NTC et/ou dudit matériau à base de graphène à l'aide de groupes de surface ;
b. comprenant en outre la sélection dudit au moins un groupe de surface à partir du groupe constitué de groupes oxygène, de groupes azote et d'une combinaison de ceux-ci ;
c. comprenant en outre la fourniture de ladite composition en tant que matériau adhésif ;
d. comprenant en outre la fourniture de ladite composition pour faciliter l'adhérence à l'intérieur du tissu, entre différents tissus ou entre le tissu et d'autres structures biocompatibles ;
e. comprenant en outre le mélange de ladite composition avec une composition pharmaceutique.

14. Procédé selon la revendication 10, dans lequel l'une des caractéristiques suivantes est présente :
a. comprenant en outre la sélection desdits nanotubes de carbone dans le groupe constitué par un nanotube à paroi simple (SWNT), un nanotube à double paroi (DWNT), un nanotube de carbone à parois multiples (MWCNT) et une combinaison de ceux-ci ;
b. ladite étape de fourniture de nanotubes de carbone comprend la fourniture de nanotubes de carbone ayant une longueur moyenne comprise entre 0,05 µm et 100 µm ;
c. ladite étape de fourniture de nanotubes de carbone comprend la fourniture de nanotubes de carbone ayant un diamètre moyen (OD) inférieur à 1 nm ;
d. ladite étape de fourniture de nanotubes de carbone comprend la fourniture de nanotubes de carbone ayant un diamètre moyen (OD) compris entre 1 nm et 100 nm ; et
e. ladite étape de fourniture de nanotubes de carbone comprend la fourniture de nanotubes de carbone ayant une pureté de carbone supérieure à 60%.

15. Procédé selon la revendication 10, dans lequel l'une des caractéristiques suivantes est présente :
a. comprenant en outre le mélange desdits nanotubes de carbone avec des structures de graphène dans des rapports allant de 0,01% à 99,99% ; et
b. comprenant en outre la sonication dudit complexe pour améliorer la dispersion des nanomatériaux de carbone.
